(19) Europäisches Patentamt
European Patent Office
Office européen des brevets

(11) **EP 3 200 848 B1**

(12) **EUROPÄISCHE PATENTSCHRIFT**

(45) Veröffentlichungstag und Bekanntmachung des Hinweises auf die Patenterteilung:
**28.10.2020 Patentblatt 2020/44**

(21) Anmeldenummer: **15771510.3**

(22) Anmeldetag: **29.09.2015**

(51) Int Cl.:
**A61M 1/36** *(2006.01)*

(86) Internationale Anmeldenummer:
**PCT/EP2015/001925**

(87) Internationale Veröffentlichungsnummer:
**WO 2016/050353 (07.04.2016 Gazette 2016/14)**

(54) **VERFAHREN ZUM SPÜLEN EINES DIALYSATORS**

METHOD FOR RINSING A DIALYZER

PROCÉDÉ DE LAVAGE D'UN DIALYSEUR

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **30.09.2014 DE 102014014535**

(43) Veröffentlichungstag der Anmeldung:
**09.08.2017 Patentblatt 2017/32**

(73) Patentinhaber: **Fresenius Medical Care Deutschland GmbH
61352 Bad Homburg (DE)**

(72) Erfinder: **KOPPERSCHMIDT, Pascal
97456 Dittelbrunn (DE)**

(74) Vertreter: **Herrmann, Uwe
Lorenz Seidler Gossel
Rechtsanwälte Patentanwälte
Partnerschaft mbB
Widenmayerstraße 23
80538 München (DE)**

(56) Entgegenhaltungen:
DE-A1-102008 005 516    DE-A1-102010 032 980
DE-C1- 10 128 278    US-A1- 2004 079 688
US-A1- 2014 217 027

**Beschreibung**

Die vorliegende Erfindung betrifft ein Verfahren und eine Vorrichtung zum Spülen eines Dialysators. Die Erfindung betrifft des Weiteren ein Blutbehandlungsgerät mit einer derartigen Vorrichtung.

Aus dem Stand der Technik bekannte Dialysatoren zur Durchführung einer Blutbehandlung weisen im Allgemeinen eine dialysatseitige Kammer und eine blutseitige Kammer auf, die durch eine Vielzahl von Hohlfasermembranen voneinander getrennt sind. Dabei durchströmt das Blut im Rahmen der Blutbehandlung das Innere der Hohlfasern, während der diese umgebende Bereich des Dialysators von einer Dialyselösung umströmt wird. Beispielsweise ist aus der Druckschrift US2014/0217027 eine herkömmliche Blutbehandlungsvorrichtung bekannt.

Üblicherweise weisen die Dialysatoren sowohl für die dialysatseitige Kammer als auch für die blutseitige Kammer jeweils einen Einlass und einen Auslass auf. Durch diese Ein- bzw. Auslässe wird Blut bzw. eine Dialyselösung dem Dialysator zugeführt bzw. aus diesem abgeführt.

Einmal-Dialysatoren sind in der Regel steril und trocken verpackt. Vor Beginn einer Dialysebehandlung müssen diese sowohl auf der Blutseite als auch dialysatseitig gespült werden, um Luft aus den blutseitigen und dialysatseitigen Kammern zu entfernen. Während des Spülens des extrakorporalen Kreislaufes, d.h. des Blutkreislaufes vor Beginn einer Dialysebehandlung wird das noch trockene Schlauchsystem mit Dialyselösung oder Kochsalz über das arterielle Schlauchsegment gefüllt und gespült. Die dabei entweichende Luft aus dem Schlauchsystem bzw. aus der blutseitigen Kammer des Dialysators wird in einen Leerspülbeutel oder über einen entsprechenden Port des Dialysesystems bzw. des Blutbehandlungsgerätes über das venöse Schlauchsystem abgeführt. Die blutseitige Kammer des Dialysators wird somit über das konnektierte extrakorporale Schlauchsystem entlüftet, das während der Behandlung mit dem Patienten in Verbindung steht und durch das Blut vom Patienten zum Dialysator und zurück vom Dialysator zum Patienten geleitet wird.

Die dialysatseitige Kammer des Dialysators wird über die konnektierte Hydraulik, d.h. über den dialysatseitigen Kreislauf bzw. dessen Komponenten entlüftet. Dieser dialysatseitige Kreislauf weist zumindest eine Leitung auf, die mit dem Einlass der dialysatseitigen Kammer in Verbindung steht sowie wenigstens eine Leitung, die mit dem Auslass der dialysatseitigen Kammer des Dialysators in Verbindung steht. Durch diese Leitungen wird im Betrieb des Blutbehandlungsgerätes die Dialyselösung zu dem Dialysator und von dem Dialysator transportiert.

Möglich ist es auch, die Luft in der dialysatseitigen Kammer über die Membran zu transportieren, sofern die Membran noch nicht benetzt ist.

Zur Entlüftung des Dialysators kann die dialysatseitige Kammer des Dialysators vor der blutseitigen Kammer entlüftet werden oder umgekehrt.

Während der Spülung des Dialysators ist es nicht immer möglich, die Zufuhr der Spülflüssigkeit entsprechend dem Auftrieb der Luft an dem tiefergelegenen Konnektor des Dialysators durchzuführen. Die Spülung erfolgt somit in der Dialysatorkammer entgegen der Gravitation von oben nach unten. Hierbei wird das verdrängte Volumen mit dem geförderten Volumen mitgerissen.

Die Dauer der Spülung und die Menge des Spülvolumens hängen von zahlreichen Parametern ab. Ein größeres Kammervolumen des Dialysators und ein geringerer Volumenstrom der Spülflüssigkeit erhöhen die zur Entlüftung nötige Spüldauer. Bei derzeit bekannten Verfahren zur Entlüftung wird ein fest vorgegebenes Spülvolumen verwendet und es wird davon ausgegangen, dass die Entlüftung des Dialysators vollzogen ist, wenn dieses Spülvolumen durch den Dialysator hindurchgeleitet wurde. Auch ist es bekannt, eine vorgegebene Zeit, z.B. fünf Minuten zu spülen und dann davon auszugehen, dass eine Entlüftung nach Ablauf dieser Zeitspanne erfolgt ist.

Ein Nachteil bei den bekannten Vorgehensweisen besteht darin, dass mitunter eine vollständige Entleerung des Dialysators von Luft nicht immer gegeben ist. Eine Prüfung der verbleibenden Luftmenge in der dialysatseitigen Kammer des Dialysators erfolgt bei aus dem Stand der Technik bekannten Verfahren nicht. Andererseits kann der Fall eintreten, dass bei bekannten Verfahren länger gespült wird oder ein größeres Spülvolumen verwendet wird, als dies zur Entlüftung des Dialysators an sich notwendig wäre.

Der vorliegenden Erfindung liegt daher die Aufgabe zugrunde, ein Verfahren der eingangs genannten Art weiterzubilden, dass der Spülvorgang hinsichtlich der Menge der Spülflüssigkeit und/oder hinsichtlich der Dauer des Spülvorgangs gegenüber bekannten Vorgehensweisen optimiert wird.

Diese Aufgabe wird durch ein Verfahren mit den Merkmalen des Einspruchs 1 gelöst. Danach ist vorgesehen, dass am Auslass des Dialysators oder stromabwärts des Dialysators in dem dialysatseitigen Kreislauf wenigstens eine Eigenschaft der Spülflüssigkeit gemessen wird, um einen oder mehrere auslassseitige Messwerte zu erhalten, wobei die gemessene Eigenschaft von der Menge der Luft in der Spülflüssigkeit abhängt.

Bei dieser gemessenen Eigenschaft kann es sich beispielsweise um die Leitfähigkeit der Spülflüssigkeit, insbesondere um die elektrische Leitfähigkeit der Spülflüssigkeit, um die Schallgeschwindigkeit, mit der sich Schall in der Spülflüssigkeit ausbreitet oder auch um eine optische Eigenschaft der Spülflüssigkeit handeln. Auch andere Eigenschaften bzw. Parameter, die von der Luftmenge der Spülflüssigkeit abhängen, sind für das Verfahren geeignet.

Besonders vorteilhaft ist es, wenn die gemessene Eigenschaft die Leitfähigkeit der Spülflüssigkeit darstellt. Im hydraulischen System von Blutbehandlungsgeräten, d.h. im Dialysekreislauf befinden sich üblicherweise ohnehin Leitfähigkeitssensoren, die für das vorliegende Verfahren genutzt werden können.

Eine bevorzugte Ausgestaltung der Erfindung besteht somit darin, dass über das hydraulische System eines Blutbehandlungsgerätes, d.h. über den dialysatseitigen Kreislauf Luft aus der dialysatseitigen Kammer des Dialysators entleert wird und diese über das hydraulische System mit der Spülflüssigkeit abtransportiert wird. Im dialysatseitigen Kreislauf befinden sich üblicherweise Leitfähigkeitssensoren bzw. - zellen, die im Behandlungsbetrieb der Dialyse die Leitfähigkeit der Dialyselösung überwachen. Die an den Leitfähigkeitszellen vorbeiströmende Luft führt zu einer deutlichen Reduktion der in der Spülflüssigkeit bestimmten Leitfähigkeit des Luft-Flüssigkeit-Gemisches. Mittels der Leitfähigkeitszellen bzw. anderer Sensoren, mittels derer eine Eigenschaft der Spülflüssigkeit, die von der Luftmenge abhängt, gemessen werden kann, lässt sich die Leitfähigkeit bzw. die Eigenschaft des Luft-Flüssigkeit-Gemisches während des Spülprozesses des Dialysators bestimmen und es lässt sich prüfen, ob der Luftanteil in der Spülflüssigkeit bzw. in dem Gemisch aus Luft und der Spülflüssigkeit reduziert wird, woraus auf eine ausweichende Luftentleerung der dialysatseitigen Kammer des Dialysators geschlossen werden kann.

Auf der Grundlage des oder der auslassseitigen Messwerte kann dann der Spülvorgang selbsttätig durch das Gerät oder manuell beendet werden und/oder es kann signalisiert werden, dass der Spülvorgang beendet ist.

Gemäß der Erfindung ist vorgesehen, dass am Einlass des Dialysators oder stromaufwärts des Dialysators in dem dialysatseitigen Kreislauf ebenfalls wenigstens eine Eigenschaft der Spülflüssigkeit gemessen wird, um einen oder mehrere einlassseitige Messwerte zu erhalten, wobei die Eigenschaft von der Menge der Luft in der Spülflüssigkeit abhängt und wobei der oder die einlassseitigen Messwerte mit dem oder den auslassseitigen Messwerten verglichen werden.

Es handelt sich bei der auslassseitig gemessenen Eigenschaft um dieselbe Eigenschaft wie bei der einlassseitig gemessenen Eigenschaft, d.h. beispielsweise um die Leitfähigkeit der Spülflüssigkeit etc.

Liegen die einlassseitigen und auslassseitigen Messwerte nahe beieinander bzw. stimmen diese überein, kann darauf rückgeschlossen werden, dass sich keine Luft mehr in der dialysatseitigen Kammer befindet und der Spülvorgang daher beendet wird oder zumindest entsprechend signalisiert wird, dass dieser beendet werden kann.

Denkbar ist es, dass der Spülvorgang beendet wird oder eine ausreichende Spülung signalisiert wird oder auf eine solche ausreichende Spülung geschlossen wird, wenn die auslassseitigen Messwerte konstant sind oder in einem bestimmten Toleranzbereich liegen. Zu Beginn des Spülvorganges zeigen die auslassseitigen Messwerte starke Schwankungen. So ist die Leitfähigkeit des Luft-Flüssigkeit-Gemisches, das zu Beginn des Spülvorgangs aus der dialysatseitigen Kammer strömt nahe Null oder bei null und stabilisiert sich erst nach einer bestimmten Spüldauer. Dies ist darauf zurückzuführen, dass es eine gewisse Zeit in Anspruch nimmt, bis die Luft aus der dialysatseitigen Kammer vollständig verdrängt ist, sodass auslassseitig schließlich die Leitfähigkeit oder eine sonstige Eigenschaft der weitgehend oder vollständig luftfreien Spülflüssigkeit gemessen wird.

Denkbar ist es weiterhin, dass der Spülvorgang beendet wird oder eine ausreichende Spülung signalisiert oder auf diese geschlossen wird, wenn die Standardabweichung der auslassseitigen Messwerte unterhalb eines bestimmten Grenzwertes liegt oder einen bestimmten Grenzwert nicht überschreitet oder innerhalb eines bestimmten Toleranzbereiches liegt. Ist die Standardabweichung der gemessenen Werte gering, kann darauf rückgeschlossen werden, dass die auslassseitigen Messwerte eine hinreichende Stabilität haben und dass eine weitgehende oder vollständige Entlüftung der dialysatseitigen Kammer stattgefunden hat.

In einer weiteren Ausgestaltung der Erfindung ist vorgesehen, dass der Spülvorgang beendet wird oder eine ausreichende Spülung oder auf eine solche geschlossen wird, wenn der Erwartungswert der auslassseitigen Messwerte innerhalb eines bestimmten Toleranzbereiches liegt und/oder wenn die Differenz der Erwartungswerte der auslassseitigen und der einlassseitigen Messwerte unterhalb eines bestimmten Grenzwertes liegen oder einen bestimmten Grenzwert nicht überschreiten. Der Begriff "Erwartungswert" ist vorzugsweise als Mittelwert über eine Mehrzahl von Messwerten zu verstehen, die vorzugsweise in einem zeitlich gleitenden Fenster erfasst werden.

Denkbar ist es, dass der Erwartungswert der auslassseitigen Messwerte innerhalb eines bestimmten Toleranzbereiches liegt - bei der Messung der Leitfähigkeit z.B. in einem Toleranzbereich der Breite von 0,1 mS/cm. Denkbar ist es auch, dass die Differenz der Erwartungswerte einlassseitig und auslassseitig bestimmt wird und überprüft wird, ob diese in einem bestimmten Toleranzbereich liegt.

In einer weiteren Ausgestaltung der Erfindung ist vorgesehen, dass der Quotient aus der Differenz der gemessenen Eigenschaft und derselben Eigenschaft bei vollständig belüfteter dialysatseitiger Kammer und der Differenz aus dieser Eigenschaft bei vollständig entlüfteter und vollständig belüfteter Eigenschaft bestimmt wird. Auf der Grundlage dieses Wertes kann der Spülvorgang dann beendet werden oder eine ausreichende Spülung signalisiert oder auf diese geschlossen werden, je nachdem welchen Wert der Quotient annimmt.

In weiterer Ausgestaltung der Erfindung ist vorgesehen, dass der Volumenstrom, mit der die Spülflüssigkeit durch die dialysatseitige Kammer strömt, zeitlich konstant oder auch zeitlich variabel ist. Eine zeitlich variable Förderrate ist vorteilhaft, um mit Hilfe auftretender Turbulenzen in der Dialysatorkammer das Mitreißen von Luftblasen zu erleichtern.

Die vorliegende Erfindung betrifft des Weiteren eine Vorrichtung mit den Merkmalen des Anspruchs 8. Darin ist vorgesehen, dass am Auslass des Dialysators oder stromabwärts des Dialysators in dem dialysatseitigen Kreislauf wenigstens ein Sensor angeordnet ist, der die genannte Eigenschaft der Spülflüssigkeit misst, wie beispielsweise eine Leitfähigkeit oder eine sonstige Eigenschaft, die von der Menge der Luft in der Spülflüssigkeit abhängt.

Bei der Eigenschaft kann es sich wie ausgeführt um die Schallgeschwindigkeit, die Leitfähigkeit oder auch um eine optische Eigenschaft der Spülflüssigkeit handeln. Die Vorrichtung weist vorzugsweise eine Auswerteeinheit auf, die derart ausgebildet ist, dass diese auf der Grundlage des oder der gemessenen Werte den Spülvorgang beendet und/oder signalisiert, dass dieser beendet werden kann.

Zusätzlich ist auch am Einlass des Dialysators oder stromaufwärts des Dialysators wenigstens ein weiterer Sensor angeordnet, der dieselbe Eigenschaft der Spülflüssigkeit einlassseitig misst. Des Weiteren ist wenigsten eine Auswerteeinheit vorgesehen, die einen Vergleich zwischen dem oder den einlassseitigen Messwerten und dem oder den auslassseitigen Messwerten vornimmt.

Diese Auswerteeinheit ist derart ausgebildet, dass diese den Spülvorgang beendet oder dessen Beendigung veranlasst oder eine ausreichende Spülung signalisiert, wenn die auslassseitigen Messwerte konstant sind oder in einem bestimmten Toleranzbereich liegen.

Die Auswerteeinheit kann derart ausgebildet sein, dass der Spülvorgang beendet wird oder eine ausreichende Spülung signalisiert wird, wenn die Standardabweichung der auslassseitigen Messwerte unterhalb eines bestimmten Grenzwertes liegt oder einen bestimmten Grenzwert nicht überschreitet oder innerhalb eines bestimmten Toleranzbereiches liegt.

Die Auswerteeinheit kann des Weiteren derart ausgebildet sein, dass der Spülvorgang beendet wird bzw. deren Beendigung veranlasst wird oder eine ausreichende Spülung signalisiert wird, wenn der Erwartungswert der auslassseitigen Messwerte innerhalb eines bestimmten Toleranzbereiches liegt und/oder wenn die Differenz der Erwartungswerte der auslassseitigen und der einlassseitigen Messwerte unterhalb eines bestimmten Grenzwertes liegt oder einen bestimmten Grenzwert nicht überschreitet.

In einer weiteren Ausgestaltung der Erfindung ist vorgesehen, dass wenigstens eine Auswerteeinheit vorgesehen ist, die so ausgeführt ist, dass der Quotient aus der Differenz der gemessenen Eigenschaften und dieser Eigenschaft bei vollständig belüfteter dialysatseitiger Kammer und der Differenz aus der Eigenschaft bei vollständig entlüfteter und vollständig belüfteter Kammer bestimmt wird und auf der Grundlage des Wertes dieses Quotienten der Spülvorgang beendet wird oder eine ausreichende Spülung signalisiert wird.

Weiterhin kann vorgesehen sein, dass die Vorrichtung zumindest eine Pumpe aufweist, die so ausgebildet ist, dass diese die Spülflüssigkeit durch die dialysatseitige Kammer fördert, wobei diese Pumpe so ausgebildet sein kann oder betrieben wird, dass die Förderrate der Spülflüssigkeit zeitlich konstant oder zeitlich variabel ist.

Die Erfindung betrifft des Weiteren ein Blutbehandlungsgerät insbesondere ein Dialysegerät mit wenigstens einer Vorrichtung gemäß einen der Ansprüche 9-16.

Es wird darauf hingewiesen, dass der Begriff "Dialysator" nicht zwingend auf einen Dialysator beschränkt ist, der im Rahmen einer Hämodialysebehandlung eingesetzt wird. Der Begriff umfasst auch Filter, die bei anderen Blutbehandlungsverfahren, wie beispielsweise bei der Hämodiafiltration zum Einsatz kommen.

Weitere Einzelheiten und Vorteile der Erfindung werden anhand eines in der Zeichnung dargestellten Ausführungsbeispiels näher erläutert. Es zeigen:

Figur 1:     Eine schematische Ansicht eines Dialysators mit dem dialysatseitigen Kreislauf sowie Leitfähigkeitssensoren in der dialysatseitigen Zuleitung sowie in der dialysatseitigen Ableitung,

Figur 2:     Der zeitliche Verlauf der Leitfähigkeit, gemessen mittels der Sensoren gemäß Figur 1 während des Spülvorgangs und

Figur 3:     Der zeitliche Verlauf der Leitfähigkeiten gemessen mit den Sensoren gemäß Figur 1 während des Spülvorgangs bei ausreichender Entlüftung.

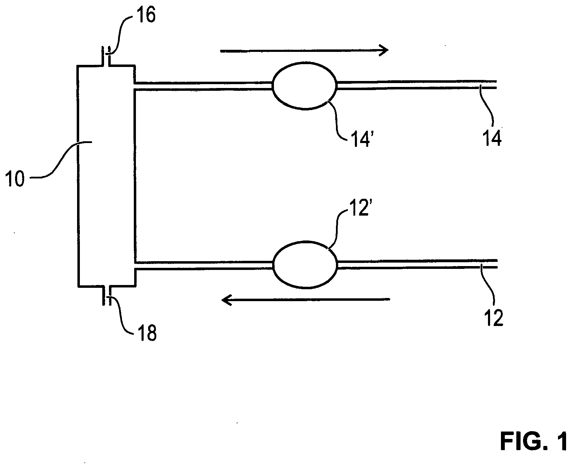

Figur 1 zeigt einen Dialysator 10 sowie einen Teil des hydraulischen Systems bzw. des dialysatseitigen Kreislaufes in Form der Zuleitung 12 und der Ableitung 14. Durch diese Leitungen wird gemäß der dargestellten Pfeilrichtung Spülflüssigkeit in die dialysatseitige Kammer eingeleitet bzw. aus dieser abgeleitet. Während des Betriebes eines Blutbehandlungsgerätes, wird durch diese Leitungen Dialyselösung zu dem Dialysator hin und von dem Dialysator abgeführt.

Die beiden Leitungen 12, 14 stehen mit einer dialysatseitigen Kammer des Dialysator in Fluidverbindung. Diese ist von einer blutseitigen Kammer des Dialysators durch eine oder mehrere Membrane, vorzugsweise durch ein Hohlfaserbündel getrennt. Die blutseitigen Zu- bzw. Abläufe des Dialysators, die mit der blutseitigen Kammer in Verbindung stehen sind in Figur 1 mit dem Bezugszeichen 16 und 18 gekennzeichnet.

Wie dies aus Figur 1 hervorgeht, befindet sich sowohl in der Zuleitung 12 sowie auch in der Ableitung 14 jeweils eine Leitfähigkeitsmesszelle 12', 14', die die Leitfähigkeit der Spülflüssigkeit vor dem Dialysator 10 und auch nach dem Dialysator 10 messen. Im Behandlungsbetrieb dienen diese Leitfähigkeitssensoren 12', 14' zur Messung der Leitfähigkeit der Dialyselösung.

Mittels der Leitfähigkeitszellen 12', 14' lässt sich die Leitfähigkeit des Luft-Flüssigkeit-Gemisches während des Spülprozesses des Dialysators bestimmen und prüfen, ob sich der Luftanteil im Gemisch reduziert, woraus auf eine ausreichende Luftentleerung der dialysatseitigen Kammer des Dialysators 10 geschlossen werden kann.

Die Messung der Leitfähigkeit bzw. einer sonstigen Eigenschaft, die von der Anwesenheit von Luft in der Dialyselösung bzw. Spülflüssigkeit abhängt wird entweder zeitlich kontinuierlich oder zu mehreren Zeitpunkten ermittelt.

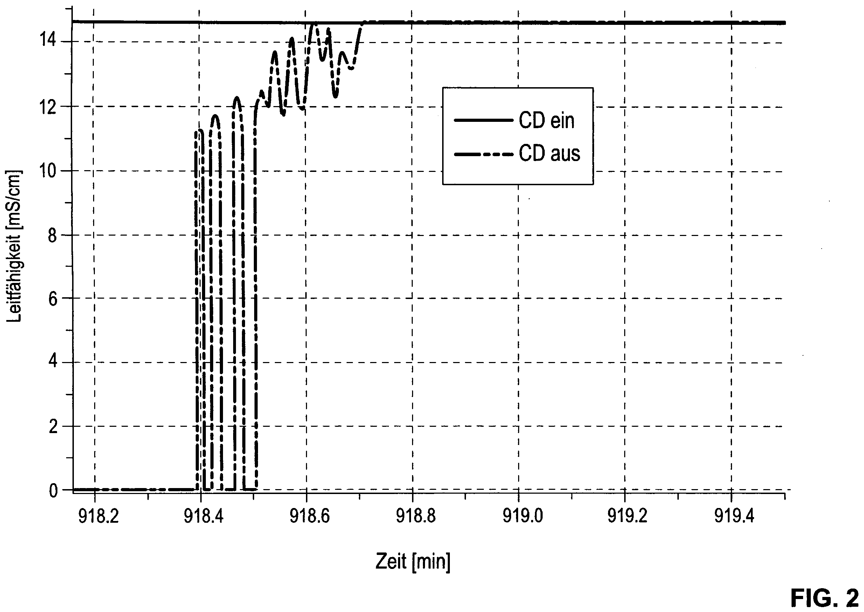

Figur 2 zeigt die Leitfähigkeiten der in die dialysatseitigen Kammer des Dialysators 10 einfließenden Spülflüssigkeit (CD ein) sowie der aus der dialysatseitigen Kammer der Dialysators 10 ausfließenden Spülflüssigkeit (CD aus) während des Spülvorganges.

Wie dies aus Figur 2 hervorgeht, zeigt die Leitfähigkeit der in die Dialysatorkammer hineinfließenden Spülflüssigkeit einen konstanten Wert von 14,6 mS/cm, während die Leitfähigkeit des aus dem Dialysator fließenden Luft-Flüssigkeit-Gemisches zuerst keine Leitfähigkeit und dann deutliche Schwankungen aufweist. Erst nach einer bestimmten Spüldauer stabilisiert sich die Leitfähigkeit der aus dem Dialysator abfließenden Spülflüssigkeit, da die Menge der Luft, die mit der Spülflüssigkeit mitgerissen wird im Laufe des Spülvorgangs abnimmt.

Vorzugsweise in Verbindung mit einer variablen Volumenrate zur effizienten Spülung der Dialysatorkammer lässt sich anhand der Analyse der Leitfähigkeiten oder eines sonstigen geeigneten Parameters der ein- bzw. ausfließenden Spülflüssigkeit der Status des Dialysatorfüllens bzw. der Luftentfernung prüfen.

Wie dies aus Figur 2 hervorgeht, gleichen sich gegen Ende des Spülvorgangs die Leitfähigkeiten einströmseitig und ausströmseitig an.

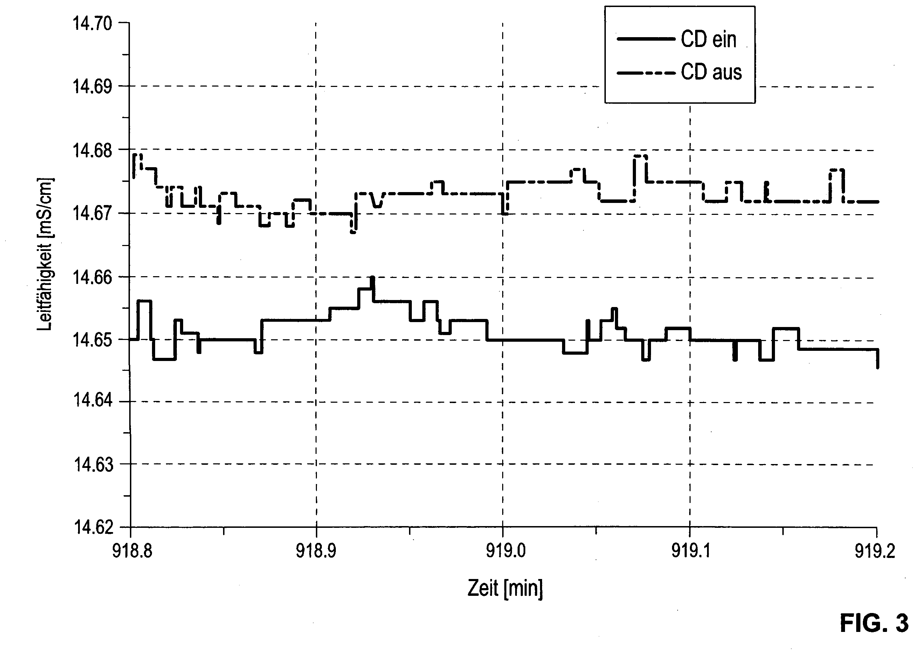

Figur 3 zeigt den Verlauf der Leitfähigkeiten wie in Figur 2 ab einem Zeitpunkt, in dem die Füllung erfolgreich geschlossen ist bzw. die Luftentleerung erfolgt ist. Wie dies aus Figur 3 hervorgeht, ergibt sich, dass die Schwankungen auslassseitig gering sind und darüber hinaus, dass die Differenz zwischen den einlassseitigen und den auslassseitigen Messwerten ebenfalls gering ist.

Das Spülverfahren findet vorzugsweise bei variabler Spülvolumenförderung, d.h. bei variabler Förderrate der Spülflüssigkeit zur Generierung von Turbulenzen im Dialysator statt. Von der Erfindung ist jedoch auch eine konstante Förderrate umfasst.

In einer denkbaren Ausführungsform der Erfindung ist vorgesehen, dass die Spülprozedur beendet wird oder entsprechend signalisiert wird, dass sie beendet werden kann, wenn im ausfließenden Luft-Flüssigkeit-Gemisch bzw. wenn in der ausfließenden Spülflüssigkeit der auslassseitige Messwert weitgehend dem einlassseitigen Messwert entspricht. Bei erfolgreicher Füllprozedur wird keine Luft mehr aus dem Dialysator transportiert, sodass die Leitfähigkeit der aus dem Dialysator strömenden Flüssigkeit stabil ist. Darunter kann verstanden werden, dass eine gegenüber dem statistischen Erwartungswert kleine Standardabweichung vorliegt. Die Standardabweichung eignet sich als Qualitätsparameter für eine Bewertung der erfolgreichen Entlüftung. Die Standardabweichung kann unter Berücksichtigung eines Erwartungswertes $X_C$ bestimmt werden.

Es erweist sich als sinnvoll, den Erwartungswert $X_C$ und eine Standardabweichung $\sigma_c$ innerhalb eines gleitenden Fensters mit N Stützelementen bzw. Messpunkten sowohl für die einströmende als auch für die ausströmende Flüssigkeit zu berechnen:

$$\langle X_c \rangle = \frac{1}{N}\sum_{i=1}^{N} c_i \qquad ; \qquad \sigma_C = \frac{1}{N}\sqrt{\sum_{i=1}^{N}\left|\langle X_c \rangle - c_i\right|^2}$$

Die Erwartungswerte der Leitfähigkeit der ein- und ausströmenden Spülflüssigkeit sollten innerhalb eines Toleranzbereiches, beispielsweise in der Größe von 0,1 mS/cm übereinstimmen. Auch die Abweichungen $\sigma_C$, die ein Maß für die Stabilität der Leitfähigkeit über die Zeit sind, sollten im Vergleich der ein- bzw. ausströmenden Flüssigkeit innerhalb eines engen Bereiches (z.B. 0,05 mS/cm) liegen. D.h. die beiden Standardabweichungen der einlassseitigen und auslassseitigen Messwerte sollten ebenfalls übereinstimmen bzw. deren Differenz innerhalb des genannten Toleranzbereiches liegen.

Die Entlüftungsprozedur kann abgebrochen werden, wenn eine bestimmte Güte der Entlüftung erreicht ist. Dies kann dann der Fall sein, wenn sowohl die Erwartungswerte als auch die Abweichungen der Leitfähigkeit der ein- bzw. ausströmenden Spülflüssigkeit innerhalb eines durch die Güte definierten Toleranzbereiches liegen.

Diese Ausführungen gelten selbstverständlich nicht nur für die Leitfähigkeit sondern auch für jeden beliebigen anderen Messparameter, der ein Maß für die Anwesenheit von Luft in der Spülflüssigkeit darstellt.

Wie eingangs ausgeführt, sind neben der Detektion von Luftsegmenten in der leitfähigen Flüssigkeit mittels Leitfähigkeitssensoren auch alternative Methoden zur Detektion einsetzbar. In Betracht kommt beispielsweise die Messung der Schallgeschwindigkeit, die in Luft und Flüssigkeit deutlich unterschiedlich ist. Somit kann als Sensor beispielsweise auch ein Ultraschalldetektor verwendet werden, der alternativ oder zusätzlich zu Leitfähigkeitssensoren eingesetzt wird.

Denkbar ist es, dass beispielsweise innerhalb eines bestimmten, den Schallsensor durchströmenden Volumens die Schalllaufzeit von Schallpulsen analysiert wird und das Verhältnis der ermittelten mittleren Laufzeit v abzüglich der Schalllaufzeit in vollständig belüfteten Laufstrecken $v_{Luft}$ zu der Laufzeitdifferenz zwischen vollständig belüfteten und vollständig entlüfteten Laufstrecken $v_{Flüssig}$ gesetzt wird:

$$\Phi[\%] = \frac{v - v_{Luft}}{v_{Flüssig} - v_{Luft}} x100$$

Dieses Verhältnis in Prozent ist ein Maß für den Grad der Entlüftung des Dialysators. Beträgt das Verhältnis 100%, entspricht die ermittelte mittlere Laufzeit v der Laufzeit bei vollständig entlüftete Laufstrecke $v_{Flüssig}$, d.h. es kann darauf rückgeschlossen werden, dass eine vollständige Entlüftung erfolgt ist.

Auch diese Vorgehensweise bezieht sich selbstverständlich nicht nur auf die Schallgeschwindigkeit als Messgröße, sondern ist auch für jede beliebige andere Messgröße anwendbar.

Neben Schallanalysen sind auch optische Analysen des Grades der Entlüftung denkbar. Da sich die Dielektrizitäten zwischen wässrigen Lösungen und Luft deutlich für viele optische Frequenzen unterscheiden, kann auch mittels optischer Messmethoden der Grad der Entlüftung bestimmt werden und daraus eine Entscheidung gefällt werden, dass der Spülvorgang abgeschlossen werden kann bzw. beendet wird.

## Patentansprüche

1. Verfahren zum Spülen eines Dialysators mit einer Spülflüssigkeit, wobei der Dialysator in einem dialysatseitigen Kreislauf eines Blutbehandlungsgerätes angeordnet ist und eine von der Spülflüssigkeit durchströmte, dialysatseitige Kammer aufweist, die einen Einlass und einen Auslass für die Spülflüssigkeit aufweist, wobei
am Auslass des Dialysators oder stromabwärts des Dialysators in dem dialysatseitigen Kreislauf eine von der Menge der Luft in der Spülflüssigkeit abhängende Eigenschaft der Spülflüssigkeit gemessen wird, um einen oder mehrere auslassseitige Messwerte zu erhalten, und
am Einlass des Dialysators oder stromaufwärts des Dialysators ebenfalls eine von der Menge der Luft in der Spülflüssigkeit abhängende Eigenschaft der Spülflüssigkeit gemessen wird, um einen oder mehrere einlassseitige Messwerte zu erhalten, **dadurch gekennzeichnet, dass**
der oder die einlassseitigen Messwerte mit dem oder den auslassseitigen Messwerten verglichen werden.

2. Verfahren nach Anspruch 1, **dadurch gekennzeichnet, dass** die Leitfähigkeit der Spülflüssigkeit oder die Schallgeschwindigkeit, mit der sich Schall in der Spülflüssigkeit ausbreitet oder eine optische Eigenschaft der Spülflüssigkeit gemessen wird.

3. Verfahren nach einem der vorhergehenden Ansprüche, **dadurch gekennzeichnet, dass** der Spülvorgang beendet wird oder eine ausreichende Spülung signalisiert oder auf diese geschlossen wird, wenn die auslassseitigen Messwerte konstant sind oder in einem bestimmten Toleranzbereich liegen.

4. Verfahren nach einem der vorhergehenden Ansprüche, **dadurch gekennzeichnet, dass** der Spülvorgang beendet wird oder eine ausreichende Spülung signalisiert oder auf diese geschlossen wird, wenn die Standardabweichung der auslassseitigen Messwerte unterhalb eines bestimmten Grenzwertes liegt oder einen bestimmten Grenzwert nicht überschreitet oder innerhalb eines bestimmten Toleranzbereichs liegt.

5. Verfahren nach einem der vorhergehenden Ansprüche, **dadurch gekennzeichnet, dass** der Spülvorgang beendet wird oder eine ausreichende Spülung signalisiert oder auf diese geschlossen wird, wenn der Erwartungswert der auslassseitigen Messwerte innerhalb eines bestimmten Toleranzbereichs liegt und/oder wenn die Differenz der Erwartungswerte der auslassseitigen und der einlassseitigen Messwerte unterhalb eines bestimmten Grenzwertes liegt oder einen bestimmten Grenzwert nicht überschreitet.

6. Verfahren nach einem der vorhergehenden Ansprüche, **dadurch gekennzeichnet, dass** der Quotient aus der Differenz der gemessenen Eigenschaft und dieser Eigenschaft bei vollständig belüfteter dialysatseitiger Kammer und der Differenz aus der Eigenschaft bei vollständig entlüfteter und vollständig belüfteter Kammer bestimmt wird und auf der Grundlage des Wertes dieses Quotienten der Spülvorgang beendet wird oder eine ausreichende Spülung signalisiert oder auf diese geschlossen wird.

7. Verfahren nach einem der vorhergehenden Ansprüche, **dadurch gekennzeichnet, dass** die Volumenrate, mit der die Spülflüssigkeit durch die dialysatseitige Kammer strömt, zeitlich konstant oder zeitlich variabel ist.

8. Vorrichtung zum Spülen eines Dialysators mit:

einer Spülflüssigkeit,
einem Dialysator, und
einem dialysatseitigen Kreislauf eines Blutbehandlungsgerätes, in dem der Dialysator angeordnet ist, wobei der Dialysator eine dialysatseitige Kammer aufweist, die einen Einlass und einen Auslass für die Spülflüssigkeit aufweist und von der Spülflüssigkeit durchströmt werden kann, und
am Auslass des Dialysators oder stromabwärts des Dialysators in dem dialysatseitigen Kreislauf ein Sensor angeordnet ist, der zum Messen einer von der Menge der Luft in der Spülflüssigkeit abhängenden Eigenschaft der Spülflüssigkeit ausgelegt ist, um einen oder mehrere auslassseitige Messwerte zu erhalten, und
am Einlass des Dialysators oder stromaufwärts des Dialysators ein weiterer Sensor angeordnet ist, der dazu ausgelegt ist, eine von der Menge der Luft in der Spülflüssigkeit abhängende Eigenschaft der Spülflüssigkeit zu messen, um einen oder mehrere einlassseitige Messwerte zu erhalten, **dadurch gekennzeichnet, dass** eine Auswerteeinheit vorgesehen ist, die dazu ausgelegt ist, den oder die einlassseitigen Messwerte mit dem oder den auslassseitigen Messwerten zu vergleichen, wobei die Auswerteeinheit derart ausgeführt ist, dass diese den Spülvorgang beendet oder eine ausreichende Spülung signalisiert, wenn die auslassseitigen Messwerte konstant sind oder in einem bestimmten Toleranzbereich liegen.

9. Vorrichtung nach Anspruch 8, **dadurch gekennzeichnet, dass** der Sensor derart ausgeführt ist, dass dieser die Leitfähigkeit der Spülflüssigkeit oder die Schallgeschwindigkeit, mit der sich Schall in der Spülflüssigkeit fortsetzt oder eine optische Eigenschaft der Spülflüssigkeit misst.

10. Vorrichtung nach einem der Ansprüche 8 bis 9, **dadurch gekennzeichnet, dass** wenigstens eine Auswerteeinheit vorgesehen ist, die derart ausgeführt ist, dass Spülvorgang beendet wird oder eine ausreichende Spülung signalisiert wird, wenn die Standardabweichung der auslassseitigen Messwerte unterhalb eines bestimmten Grenzwertes liegt oder einen bestimmten Grenzwert nicht überschreitet oder innerhalb eines bestimmten Toleranzbereichs liegt.

11. Vorrichtung nach einem der Ansprüche 8 bis 10, **dadurch gekennzeichnet, dass** wenigstens eine Auswerteeinheit vorgesehen ist, die derart ausgeführt ist, dass der Spülvorgang beendet wird oder eine ausreichende Spülung signalisiert wird, wenn der Erwartungswert der auslassseitigen Messwerte innerhalb eines bestimmten Toleranzbereichs liegt und/oder wenn die Differenz der Erwartungswerte der auslassseitigen und der einlassseitigen Messwerte unterhalb eines bestimmten Grenzwertes liegt oder einen bestimmten Grenzwert nicht überschreitet.

12. Vorrichtung nach einem der Ansprüche 8 bis 11, **dadurch gekennzeichnet, dass** wenigstens eine Auswerteeinheit vorgesehen ist, die derart ausgeführt ist, dass der Quotient aus der Differenz der gemessenen Eigenschaft und dieser Eigenschaft bei vollständig belüfteter dialysatseitiger Kammer und der Differenz aus der Eigenschaft bei vollständig entlüfteter und vollständig belüfteter Kammer bestimmt wird und auf der Grundlage des Wertes dieses Quotienten der Spülvorgang beendet wird oder eine ausreichende Spülung signalisiert wird.

13. Vorrichtung nach einem der Ansprüche 8 bis 12, **dadurch gekennzeichnet, dass** wenigstens eine Pumpe vorgesehen ist, die derart ausgebildet, dass diese die Spülflüssigkeit durch die dialysatseitige Kammer fördert, wobei die Pumpe derart ausgebildet ist, dass diese die Spülflüssigkeit mit einer zeitlich konstanten oder zeitlich variablen Förderrate durch die dialysatseitige Kammer fördert.

14. Blutbehandlungsgerät, insbesondere Dialysegerät mit wenigstens einer Vorrichtung gemäß einem der Ansprüche 8 bis 13.

## Claims

1. Method of flushing a dialyzer with a flushing liquid, wherein the dialyzer is arranged in a dialyzate-side circuit of a blood treatment device and comprises a dialyzate-side chamber which has an inlet and an outlet for the flushing liquid and which is flowed through by the flushing liquid, wherein
a property of the flushing liquid that depends on the quantity of the air in the flushing liquid is measured at the outlet of the dialyzer or downstream of the dialyzer in the dialyzate-side circuit to obtain one or more outlet-side measured values, and
a property of the flushing liquid that depends on the quantity of the air in the flushing liquid is likewise measured at the inlet of the dialyzer or upstream of the dialyzer to obtain one or more inlet-side measured values,
**characterized in that**
the inlet-side measured value or values are compared with the outlet-side measured value or values.

2. Method in accordance with claim 1, **characterized in that** the conductivity of the flushing liquid or the speed of sound at which sound propagates in the flushing liquid or an optical property of the flushing liquid is measured.

3. Method in accordance with one of the preceding claims, **characterized in that** the flushing process is ended or a sufficient flushing is signaled or a conclusion thereon is drawn when the outlet-side measured values are constant or lie in a certain tolerance range.

4. Method in accordance with one of the preceding claims, **characterized in that** the flushing process is ended or a sufficient flushing is signaled or a conclusion thereon is drawn when the standard deviation of the outlet-side measured values is below a certain limit value or does not exceed a certain limit value or lies within a certain tolerance range.

5. Method in accordance with one of the preceding claims, **characterized in that** the flushing process is ended or that a sufficient flushing is signaled or a conclusion thereon is drawn when the expected value of the outlet-side measured values lies within a certain tolerance range and/or when the difference of the expected values of the outlet-side measured values and of the inlet-side measured values is below a specific limit value or does not exceed a certain limit value.

6. Method in accordance with one of the preceding claims, **characterized in that** the quotient is determined from the difference of the measured property and of this property with a completely ventilated dialyzate-side chamber and the difference from the property with a completely vented and a completely ventilated chamber, and the flushing process is ended or a sufficient flushing is signaled or a conclusion thereon is drawn on the basis of the value of this quotient.

7. Method in accordance with one of the preceding claims, **characterized in that** the volume rate at which the flushing liquid flows through the dialyzate-side chamber is constant in time or variable in time.

8. Apparatus for flushing a dialyzer, comprising:

   a flushing liquid,
   a dialyzer, and
   a dialyzate-side circuit of a blood treatment device in which the dialyzer is arranged, wherein
   the dialyzer has at least one dialyzate-side chamber which comprises an inlet and an outlet for the flushing liquid and which can be flowed through by the flushing liquid,
   a sensor is arranged at the outlet of the dialyzer or downstream of the dialyzer in the dialyzate-side circuit, the sensor being configured to measure at least one property of the flushing liquid that depends on the quantity of the air in the flushing liquid to obtain one or more outlet-side measured values, and
   a further sensor is arranged at the inlet of the dialyzer or upstream of the dialyzer, which sensor is configured to measure a property of the flushing liquid that depends on the quantity of the air in the flushing liquid to obtain one or more inlet-side measured values,
   **characterized in that**
   an evaluation unit is provided, which is configured to compare the inlet-side measured value or values with the outlet-side measured value or values, wherein the evaluation unit is configured such that it ends the flushing process or signals a sufficient flushing when the outlet-side measured values are constant or lie in a certain tolerance range.

9. Apparatus in accordance with claim 8, **characterized in that** the sensor is configured such that it measures the conductivity of the flushing liquid or the speed of sound at which sound propagates in the flushing liquid or measures an optical property of the flushing liquid.

10. Apparatus in accordance with one of the claims 8 to 9, **characterized in that** at least one evaluation unit is provided which is configured such that the flushing process is ended or a sufficient flushing is signaled when the standard deviation of the outlet-side measured values lies below a certain limit value or does not exceed a certain limit value or lies within a certain tolerance range.

11. Apparatus in accordance with one of the claims 8 to 10, **characterized in that** at least one evaluation unit is provided which is configured such that the flushing process is ended or a sufficient flushing is signaled when the expected value of the outlet-side measured values lies within a certain tolerance range and/or when the difference of the expected values of the outlet-side and of the inlet-side measured values lies below a certain limit value or does not exceed a certain limit value.

12. Apparatus in accordance with one of the claims 8 to 11, **characterized in that** at least one evaluation unit is provided which is configured such that the quotient is determined from the difference of the measured property and of this property with a completely ventilated dialyzate-side chamber and the difference from the property with a completely vented and completely ventilated chamber and such that the flushing process is ended or a sufficient flushing is signaled on the basis of the value of this quotient.

13. Apparatus in accordance with one of the claims 8 to 12, **characterized in that** at least one pump is provided, which is configured such that it conveys the flushing liquid through the dialyzate-side chamber, wherein the pump is configured such that it conveys the flushing liquid through the dialyzate-side chamber at a conveying rate constant in time or variable in time.

14. Blood treatment device, in particular a dialysis machine, comprising at least one apparatus in accordance with one of the claims 8 to 13.

**Revendications**

1. Procédé de lavage d'un dialyseur à l'aide d'un liquide de lavage, le dialyseur étant agencé dans un circuit côté dialysat d'un dispositif de traitement du sang, et comprenant une chambre côté dialysat comportant un orifice d'entrée et un orifice de sortie pour le liquide de lavage et étant traversée par le liquide de lavage, une propriété du liquide de lavage, dépendant de la quantité d'air dans le liquide de lavage, étant mesurée au niveau de l'orifice de sortie du dialyseur ou en aval du dialyseur dans le circuit côté dialysat, afin d'obtenir une ou plusieurs valeurs mesurées côté sortie, et
une propriété du liquide de lavage, dépendant de la quantité d'air dans le liquide de lavage, étant mesurée de manière similaire au niveau de l'orifice d'entrée du dialyseur ou en amont du dialyseur, afin d'obtenir une ou plusieurs valeurs mesurées côté entrée,
**caractérisé en ce que**
la ou les valeurs mesurées côté entrée sont comparées avec la ou les valeurs mesurées côté sortie.

2. Le procédé selon la revendication 1, **caractérisé en ce que** la conductivité du liquide de lavage, ou la vitesse du son à laquelle le son se propage dans le liquide de lavage, ou une propriété optique du liquide de lavage, est mesurée.

3. Le procédé selon l'une des revendications précédentes, **caractérisé en ce que** le procédé de lavage est terminé, ou un lavage suffisant est signalé, ou une conclusion est établie à ce sujet, lorsque les valeurs mesurées côté sortie sont constantes ou se trouvent dans une certaine plage de tolérances.

4. Le procédé selon l'une des revendications précédentes, **caractérisé en ce que** le procédé de lavage est terminé, ou un lavage suffisant est signalé, ou une conclusion est établie à ce sujet, lorsque l'écart type des valeurs mesurées côté sortie est inférieur à une certaine valeur limite, ou ne dépasse pas une certaine valeur limite, ou se trouve dans une certaine plage de tolérances.

5. Le procédé selon l'une des revendications précédentes, **caractérisé en ce que** le procédé de lavage est terminé, ou un lavage suffisant est signalé, ou une conclusion est établie à ce sujet, lorsque la valeur espérée des valeurs mesurées côté sortie se trouve dans une certaine plage de tolérances, et/ou lorsque la différence des valeurs espérées des valeurs mesurées côté sortie, et des valeurs mesurées côté entrée, est inférieure à une valeur limite spécifique ou ne dépasse pas une certaine valeur limite.

**6.** Le procédé selon l'une des revendications précédentes, **caractérisé en ce qu'**un quotient est déterminé à partir de la différence de la propriété mesurée et de ladite propriété avec une chambre côté dialysat entièrement ventilée et de la différence de la propriété avec une chambre entièrement aérée et une chambre entièrement ventilée, et le procédé de lavage est terminé, ou un lavage suffisant est signalé, ou une conclusion est établie à ce sujet, en fonction de la valeur dudit quotient.

**7.** Le procédé selon l'une des revendications précédentes, caractérisé en ce le taux volumique de l'écoulement du liquide de lavage à travers la chambre côté dialysat est constant dans le temps ou variable dans le temps.

**8.** Appareil de lavage d'un dialyseur, l'appareil comprenant :

un liquide de lavage,
un dialyseur, et
un circuit côté dialysat d'un dispositif de traitement du sang dans lequel le dialyseur est agencé,
le dialyseur comprenant au moins une chambre côté dialysat comportant un orifice d'entrée et un orifice de sortie pour le liquide de lavage, ladite chambre pouvant être traversée par le liquide de lavage,
un capteur étant agencé au niveau de l'orifice de sortie du dialyseur ou en aval du dialyseur dans le circuit côté dialysat, le capteur étant conçu pour mesurer au moins une propriété du liquide de lavage dépendant de la quantité d'air dans le liquide de lavage, afin d'obtenir une ou plusieurs valeurs mesurées côté sortie, et
un capteur supplémentaire est agencé au niveau de l'orifice d'entrée du dialyseur ou en amont du dialyseur, ledit capteur étant conçu pour mesurer une propriété du liquide de lavage dépendant de la quantité d'air dans le liquide de lavage, afin d'obtenir une ou plusieurs valeurs mesurées côté entrée,
**caractérisé en ce que**
une unité d'évaluation est ménagée et conçue pour comparer la ou les valeurs mesurées côté entrée avec la ou les valeurs mesurées côté sortie, l'unité d'évaluation étant conçue pour terminer le procédé de lavage, ou pour signaler un lavage suffisant, lorsque les valeurs mesurées côté sortie sont constantes ou se trouvent dans une certaine plage de tolérances.

**9.** L'appareil selon la revendication 8, **caractérisé en ce que** le capteur est conçu pour mesurer la conductivité du liquide de lavage, ou la vitesse du son à laquelle le son se propage dans le liquide de lavage, ou une propriété optique du liquide de lavage.

**10.** L'appareil selon l'une des revendications 8 à 9, **caractérisé en ce qu'**au moins une unité d'évaluation est ménagée et conçue pour terminer le procédé de lavage, ou pour signaler un lavage suffisant, lorsque l'écart type des valeurs mesurées côté sortie est inférieur à une certaine valeur limite, ou ne dépasse pas une certaine valeur limite, ou se trouve dans une certaine plage de tolérances.

**11.** L'appareil selon l'une des revendications 8 à 10, **caractérisé en ce qu'**au moins une unité d'évaluation est ménagée et conçue pour terminer le procédé de lavage, ou pour signaler un lavage suffisant, lorsque la valeur espérée des valeurs mesurées côté sortie se trouve dans une certaine plage de tolérances, et/ou lorsque la différence des valeurs espérées des valeurs mesurées côté sortie, et des valeurs mesurées côté entrée, est inférieure à une valeur limite ou ne dépasse pas une certaine valeur limite.

**12.** L'appareil selon l'une des revendications 8 à 11, **caractérisé en ce qu'**au moins une unité d'évaluation est ménagée et conçue pour déterminer un quotient à partir de la différence de la propriété mesurée et de ladite propriété avec une chambre côté dialysat entièrement ventilée et de la différence de la propriété avec une chambre entièrement aérée et une chambre entièrement ventilée, et pour terminer le procédé de lavage, ou pour signaler un lavage suffisant, en fonction de la valeur dudit quotient.

**13.** L'appareil selon l'une des revendications 8 à 12, **caractérisé en ce qu'**au moins une pompe est ménagée et conçue pour transporter le liquide de lavage à travers la chambre côté dialysat, la pompe étant conçue pour transporter le liquide de lavage à travers la chambre côté dialysat à un taux de transport constant dans le temps ou variable dans le temps.

**14.** Dispositif de traitement du sang, en particulier une machine de dialyse, comprenant au moins un appareil selon l'une des revendications 8 à 13.

**FIG. 1**

**FIG. 2**

EP 3 200 848 B1

FIG. 3

EP 3 200 848 B1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 20140217027 A